# EUROPEAN PATENT SPECIFICATION

(11) **EP 1 528 911 B1**
(45) Date of publication and mention of the grant of the patent: **20.06.2012**
(21) Application number: 03765741.8
(22) Date of filing: 18.07.2003
(51) Int. Cl.: A61Q 11/00, A61K 8/02, A61K 8/72, A61K 8/73, A61K 8/66

(54) **Enhanced breath freshening film with protease**
Mundgeruch verbessernder Film mittels Protease
Film pour rafraichir l'haleine ameliore par protease

(30) Priority: 23.07.2002 US 200939
(43) Date of publication of application: 11.05.2005
(73) Proprietor: Colgate-Palmolive Company, New York NY 10022-7499 (US)
(72) Inventor: SZELES, Lori, H., Hillsborough, NJ 08844 (US); MOHSENI, Sonya, Stewartsville, NJ 08886 (US); XU, Guofeng, Princeton, NJ 08542 (US); WILLIAMS, Malcolm, Piscataway, NJ 08854 (US); VISCIO, David, B., Monmouth Junction, NJ 08852 (US); MASTERS, James, G., Ringoes, NJ 08551 (US)
(74) Representative: Jenkins, Peter David
(86) International application number: PCT/US2003/022530
(87) International publication number: WO 2004/009050

(56) References cited:
- EP-A- 0 524 732
- WO-A-00/18365
- WO-A-97/38670
- WO-A-03/030883
- US-A- 4 740 368
- US-A- 5 922 346

## Description

### BACKGROUND OF THE INVENTION

### 1. Field of the Invention

The present invention relates to an orally consumable film for delivering breath freshening agents to the oral cavity and in particular a consumable film having breath freshening properties enhanced by the presence of enzymes incorporated in the film.

### 2. The Prior Art

Halitosis, the technical term for breath malodor, is an undesirable condition. Breath malodor results when proteins, particles from food, and saliva debris are decomposed by mouth bacteria. The tongue, with its fissures and large, bumpy surface area, retains considerable quantities of food and debris that support and house a large bacterial population. Under low oxygen conditions, the bacteria form malodorous volatile sulfur compounds (VSC) - such as hydrogen sulfide and methyl mercaptans.

Bacteria thrive on the tongue. For the most part, the bacteria are a part of a protective bio-film that essentially renders them resistant to most treatments. Few people clean their tongue after brushing, even though it's been shown that as much as 50 percent of the mouth's bacteria can be found here. Additionally, for many people, brushing or scraping the tongue is difficult because of the gag reflex. Therefore, cleaning the tongue non-mechanically is highly desirable for those who are unable to do so with a mechanical device.

It is known to the art to use consumable water soluble or dispersible films adapted to disintegrate in the oral cavity which films contain flavoring agents for delivering breath freshening agents to mask or reduce bacteria caused breath malodor. For example, PCT application number WO 00/18365 discloses a breath freshening film adapted to dissolve in the mouth of the user, the film being comprised of a water soluble polymer such as pullulon or hydroxypropylmethyl cellulose and an essential oil selected from thymol, methyl salicylate, eucalyptol and menthol.

US 4,713,243 discloses a film for delivering therapeutic agents to the oral cavity composed of a water soluble polymer matrix of a hydroxypropyl cellulose, a homopolymer of ethylene oxide, the film having incorporated therein a pharmaceutically effective amount of medicament for the treatment of periodontal disease.

US 5,354,551 discloses a water soluble film presegmented into dosage units, the film containing conventional toothpaste ingredients and formulated with swellable polymers such as gelatin and corn starch as film forming agents which upon application to the oral cavity disintegrate, to release an active agents incorporated in the film.

US 6,177,096 discloses a film composition containing therapeutic and/or breath freshening agents for use in the oral cavity prepared from a water soluble polymer such as hydroxypropylmethyl cellulose, hydroxypropylcellulose and a polyalcohol such as glycerol, polyethylene glycol.

US-A-5,992,346 discloses a composition for reducing a free radical damage induced by tobacco products and environmental pollutants which contains reduced glutathione and a source of selenium. The specification discloses that a method for the application of the active ingredients glutathione and selenium may be incorporated in chewing gums, bubble gums, lozenges and tablets. At co. 10, lines 59-66, US-A-5,992,346 teaches that breath fresheners may be added as an optional ingredient such as amylase as disclosed in US Patent 4,740,368.

US-A-4,740,368 discloses a solid breath cleaning confection which contains the enzyme amylase which break down of halitosis forming starch food debris in the mouth.

EP-A-0524732) discloses a food or medicine containing lactic acid bacteria which are capable of staying in the oral cavity and capable of extracellular production of Dextranase which will effectively degrade dental plaque and prevent dental caries.

WO-A-97/38670 discloses an oral composition containing the enzymes Dextranase and mutanase effective for the removal of an prevention of the formation of dental plaque. Significant enzyme activity occurs in the range of pH 4 to below 6.0. Examples of an oral care product is defined at page 9, lines 10-11 of D4 as a gel, liquid, ointment or tablet.

EP patent application 02801042.0, published as WO-A-03/030883, comprises part of the state of the art under the provisions of Article 54(3) and (4) EPC and describes a rapidly dissolvable film comprising an enzyme and a water-soluble polymer.
Although the prior art water soluble consumable films have provided breath freshening benefits, the art continually seeks to enhance such benefits.

### SUMMARY OF THE INVENTION

In accordance with the present invention there is provided orally consumable film composition comprising a homogenous mixture of protease enzyme, the enzyme being dispersed in a hydrophobic diluent or dispersant, and a water soluble or dispersible film forming polymer, wherein the polymer is present in the composition from 0,1 to 5 % by weight. The film composition can deliver agents to the oral cavity effective to reduce breath malodor wherein the antimalodor efficacy of the film is significantly enhanced by incorporating a protease enzyme into the film matrix.
Enzymes are quaternary proteins and their structure, function, and stability are sensitive to processing conditions and chemical environments and often denature in such environments, for example, at elevated temperatures, that is, temperatures substantially above 45°C. It was therefore unexpected that a protease enzyme incorporated into a film matrix adapted to disintegrate in an oral cavity environment retained its proteolytic activity, during film manufacture at elevated temperatures and residence times involved in the film manufacturing process.

### DETAILED DESCRIPTION OF THE INVENTION

The film of the present invention comprises a consumable water soluble or dispersible film containing an antimalodor enzyme. The film can further comprise water, additional film forming agents, flavor agents, plasticizing agents, other antimalodor agents, emulsifying agents, coloring agents, sweeteners and fragrances.

### Enzymes

The enzymes useful in the practice of the present invention include enzymes extracted from natural fruit products well known protein substances within the class of proteases, which breakdown or hydrolyze proteins (proteases).

The proteolytic enzymes are obtained from natural sources or by the action of microorganisms having a nitrogen source and a carbon source. Examples of proteolylic enzymes useful in the practice of the present invention include papain, bromelain, chymotrypsin, ficin and alcalase. The enzymes are included in the film compositions of the present invention at a concentration of 0.1 to 5% by weight and preferably 0.2 to 2% by weight.

Papain obtained from the milky latex of the Papaya tree is the proteolytic enzyme preferred for use in the practice of the present invention and is incorporated in the film matrix of the present invention in an amount of 0.1 to 5% by weight and preferably 0.5 to 5% by weight, the papain having an activity of 150 to 939 MCU per milligram as determined by the Milk Clot Assay Test of the Biddle Sawyer Group (see J. Biol. Chem., vol. 121, pages 737-745).

Additional enzymes which may be useful in the practice of the present invention include protein substances within the class of proteases, which breakdown or hydrolyze proteins (proteases). These proteolytic enzymes are obtained from natural sources or by the action of microorganisms having a nitrogen source and a carbon source. Examples of alternative proteolylic enzymes useful in the practice of the present invention include bromelain, chymotrypsin, ficin and alcalase.

### Film Matrix

Water soluble or dispersible film forming agents used to form the film matrix of the present invention include water soluble polymers such as polyvinyl-pyrolidone, hydroxyethyl cellulose, hydroxypropyl methyl cellulose, hydroxyalkyl celluloses such as hydroxypropyl cellulose, carboxymethyl cellulose, polyvinyl alcohol, sodium alginate, guar gum, xanthan gum as well as water dispersible polymers such as polyacrylates, carboxyvinyl copolymers, methyl methacrylate copolymers and polyacrylic acid. A low viscosity hydropropylmethyl cellulose polymer (HPMC) having a viscosity in the range of about 1 to about 40 millipascal seconds (mPa·s) as determined as a 2% by weight aqueous solution of the HPMC at 20°C using a Ubbelohde tube viscometer is a preferred film matrix material. Preferably the HPMC has a viscosity of about 3 to about 20 mPa·s at 20°C such HMPC is available commercially from the Dow Chemical Company under the trade designation Methocel E5 Premium LV. Methocel E5 Premium LV is a USP grade, low viscosity HPMC having 29.1% methoxyl groups and 9% hydroxyproxyl group substitution. It is white or off-white free flowing dry powder. As a 2 weight % solution in water as measured with Ubbelohde tube viscometer it has a viscosity of 5.1 to mPa·s at 20°C.

The hydroxyalkyl methyl cellulose is incorporated in the film composition in amounts ranging from 10 to 60% by weight and preferably 15 to 40% by weight.

Cold water dispersible, swellable, physically modified and pregelatinized starches are particularly useful as texture modifier to increase the stiffness of the hydroxyalkyl methyl cellulose polymer films of the present invention. To prepare such starch products, the granular starch is cooked in the presence of water and possibly an organic solvent at a temperature not higher than 10°C higher than the gelatinization temperature. The obtained starch is then dried.

Pregelatinized corn starch is available commercially. A preferred starch is available under the trade designation Cerestar Polar Tex-Instant 12640 from the Cerestar Company. This Cerestar starch is a pregelaterized, stabilized and crosslinked waxy maize starch. It is readily dispersible and swellable in cold water. In its dry form, it is a white free flowing powder with an average particle size no greater than 180 micrometers and 85% of the particles are smaller than 75 micrometers. It has a bulk density of 441bs/ft³.

The pregelatinized starch may be incorporated in the film matrix of the present invention in an amount ranging from 5 to 50% by weight and preferably 10 to 35% by weight.

### Emulsifiers

Emulsifying agents are incorporated in the film matrix ingredients to promote homogeneous dispersion of the ingredients. Examples of suitable emulsifiers include condensation products of ethylene oxide with fatty acids, fatty alcohols, polyhyrric alcohols (e.g., sorbitan monostearate, sorbitan oleate), alkyl phenols (e.g., Tergitol) and polypropyleneoxide or polyoxybutylene (e.g., Pluronics); amine oxides such as dimethyl cocamine oxide, dimethyl lauryl amine oxide and cocoalkyldimethyl amine oxide polysorbates such as Tween 40 and Tween 80 (Hercules), glyceryl esters of fatty acid (e.g., Arlacel 186). The emulsifying agent is incorporated in the film matrix composition of the present invention at a concentration of about 0.1 to about 3% by weight and preferably about 0.2 to 1.0% by weight.

### Flavor Agents

Flavor agents that can be used to prepare the film of the present invention include those known to the art, such as natural and artificial flavors. These flavor agents may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. These flavor agents can be used individually or in admixture. Commonly used flavor include mints such as peppermint, artificial vanilla, cinnamon derivatives, and various fruit flavors, whether employed individually or in admixture. Generally, any flavoring or food additive, such as those described in Chemicals Used in Food Processing, publication 1274 by the National Academy of Sciences, pages 63-258, may be used. The amount of flavoring agent employed is normally a matter of preference subject to such factors as flavor type, individual flavor, and strength desired.

Generally the flavor agent is incorporated in the film of the present invention in an amount ranging from about 2.0 to about 30% by weight and preferably about 6 to about 25% by weight.

Sweeteners useful in the practice of the present invention include both natural and artificial sweeteners. Suitable sweetener include water soluble sweetening agents such as monosaccharides, disaccharides and plysaccharides such as xylose, ribose, glucose (dextrose), mannose, glatose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalaine methyl ester (aspartame) and sucralose.

In general, the effective amount of sweetener is utilized to provide the level of sweetness desired for a particular composition, will vary with the sweetener selected. This amount will normally be about 0.01% to about 2% by weight of the composition.

The compositions of the present invention can also contain coloring agents or colorants. The coloring agents are used in amounts effective to produce the desired color and include natural food colors and dyes suitable for food, drug and cosmetic applications. These colorants are known as FD&C dyes and lakes. The materials acceptable for the foregoing spectrum of use are preferably water-soluble, and include FD&C Blue No.2, which is the disodium salt of 5,5-indigotindisulfonic acid. Similarly, the dye known as Green No.3 comprises a 15 triphenylmethane dye and is the monosodium salt of 4-[4-N-ethyl-p-sulfobenzylarnino) diphenyl-methylene]-[1- N-ethy 1- N-sulfonium benzyl)- 2,5-cyclo-hexadienimine]. A full recitation of all FD&C and D&C dyes and their corresponding chemical structures may be found in the Kirk-Othmer Encyclopedia of Chemical Technology, Volume 5, Pages 857-884, which text is accordingly incorporated herein by reference.

Agents known to exhibit antimalodor activity can be incorporated into the film composition of the present invention including zinc gluconate, zinc citrate and/or alpha ionone. These agents function to aid in reducing mouth odor and work in combination with enzymes to reduce volatile odor causing bacterial sulfur compounds. These agents may be incorporated in the film matrix of the present invention at a concentration of about 0.1 to about 2.0% by weight and preferably about 0.15 to about 0.5% by weight.

In preparing the film composition according to the present invention, a water soluble or water dispersible film forming agent such as hydroxyalkylmethyl cellulose is dissolved in a compatible solvent such as water heated to about 60°C to about 71 °C to form a film forming composition. Thereafter, there is optionally added in the sequence, a second film forming agent such as starch, sweetener, surfactant, flavor and enzyme compound to prepare a film ingredient slurry.

The slurry is cast on a releasable carrier and dried. The carrier material must have a surface tension which allows the film solution to spread evenly across the intended carrier width without soaking to form a destructive bond between the film and the carrier substrate. Examples of suitable carrier materials include glass, stainless steel, Teflon and polyethylene impregnated paper. Drying of the film may be carried out at elevated temperatures by transversing through a zoned dryer at approximately 20-30 inches/min at temperatures ranging for example from, 70°C to 120°C, using a drying oven, drying terminal, vacuum drier, or any other suitable drying equipment for residence times which do not adversely effect the ingredients of which the film is composed.

To insure the stability of the enzyme during film manufacture and protect the enzyme tertiary protein structure, the enzyme is predispersed in a hydrophobic diluent or dispersant such as a vegetable oil, including canola oil, corn oil, peanut oil, a polyethylene glycol or a silicone oil to provide a protective shield for the enzyme during the manufacturing process.

The film once formed is segmented into dosage units by die-cutting or slitting-and-die cutting. The segmented film has a strip width and length corresponding to about the size of a postage stamp, generally about 12 to about 30 millimeter in width and about 20 to about 50 millimeters in length. The film has a thickness ranging from about 15 to about 80 micrometers, and preferably about 40 to 60 micrometers.

The film is shaped and sized to be placed in the oral cavity. The film is flexible and adheres to a surface in the mouth, usually the roof of the mouth or the tongue, and quickly dissolves, generally in less than 25-60 seconds.

The present invention is illustrated by the following examples.

### Example 1

A breath freshening film designated Composition A was prepared by using the ingredients listed in Table I below. In preparing the film, the HMPC polymer ingredient (Methocel E5LV) was added at a temperature of 70°C to 90°C, to half the amount of total deionized water used, and the solution stirred for 20 minutes at a slow speed using a IKA Labortechnik Model RW20DZMixer. The remaining amount of water maintained at room temperature (21°C) was then added and the mixing continued for 40 minutes. To this solution was added the corn starch ingredient (Cerestar Polar Tex Instant 12640) and the mixture stirred for an additional 20 minutes until the starch was completely dispersed and a homogeneous mixture was formed. To this mixture was added sucralose and mixed for 10 minutes after which the emulsifier Tween 80 was added and mixed for an additional 5 minutes. Thereafter flavor was thoroughly mixed for an additional 30 minutes to form a slurry emulsion to which as a final step the enzyme papain dispersed in canola oil was slowly added until evenly dispersed in the film ingredient slurry. The emulsion was then cast on a polyethylene coated paper substrate and passed through a 6 zone oven at a rate of 20-30 in/min and dried at 115°C to form a solid thin (40um thick) translucent film.

| **TABLE I** | |
|---|---|
| **Ingredients** | **Composition A (Wt.%)** |
| Water | 77.5 |
| HPMC | 8.55 |
| Corn starch | 4.00 |
| Flavor | 6.00 |
| Tween 80 | 0.50 |
| Canola Oil | 1.00 |
| Sucralose | 0.20 |
| Papain | 1.00 |

Human clinical studies determined that the film of Composition A significantly reduced the level of bacterial species on the tongue surface responsible for the presence of oral malodor for up to 60 minutes post application use when compared identical compositions prepared without the enzyme papain.

The evaluation of the quantity of bacteria responsible for oral malodor was determined, in-situ, in a tongue micro-flora study . The film composition was tested for its ability to reduce the micro-flora on the back of the tongue, especially those species responsible for the generation of H₂S. The study required subjects to swab one side of the back of the tongue for bacterial collection at baseline and the alternate back side of the tongue 1 hour after the first application of the Composition A film to the tongue which remained on the tongue for a time sufficient for the film to dissolve and disintegrate. The collected samples were plated onto lead acetate agar media for the selection of H₂S-forming bacteria as well as blood agar media to determine the total level of bacteria present on the tongue and incubated under anaerobic conditions at 37°C. After 72 hours, colony-forming units (CFU) of H₂S-forming bacteria, and total bacterial colony-forming units were enumerated. The mean colony forming unit results were used to calculate percent reduction from baseline.

The results of the in-vivo tongue micro-flora study are recorded in Table II below. For purposes of comparison a the procedure of Example 1 was repeated with the exception that a film composition substantially identical to Composition A (designated Composition B) was used except that papain was not present in the film composition. The antimalodor efficacy of Composition B was also assessed in the microflora test used to evaluate Composition A. These results are also recorded in Table II.

| **TABLE II** | | | | | | |
|---|---|---|---|---|---|---|
| | **Baseline** **(Mean CFU)** | | **1 Hour Post Film Application** **(Mean CFU)** | | **% Reduction** | |
| Composition | Malodor Tongue Bacteria | Total Tongue Bacteria | Malodor Tongue Bacteria | Total Tongue Bacteria | Malodor Tongue Bacteria | Total Tongue Bacteria |
| A | 4.5*10⁵ | 8.2*10⁵ | 6.9*10⁴ | 8.9*10⁴ | 84.7 | 89.2 |
| B | 1.1*10⁵ | 2.1*10⁵ | 2.2*10³ | 4.7*10⁵ | Bacterial Growth | Bacterial Growth |

The results recorded in Table II indicate that the papain containing film Composition A of the present invention, unexpectedly provided a substantially reduced quantity of tongue bacteria as compared to the comparative film Composition B which did not contain the enzyme papain.

The film Composition A of the present invention was also found to control volatile sulfur compound (VSC) formation in a clinical breath/VSC study involving the same human subjects who participated in the tongue microflora study. Breath-odor was measured using a Halimeter™ at baseline and at 1 hour after film application to the tongue. The results recorded in Table III are consistent with data represented in Table II indicating a greater reduction in breath VSC's responsible for oral malodor when compared to the comparative film Composition B in which papain enzyme was not present in the film.

| **TABLE III** | | | |
|---|---|---|---|
| **Clinical study involving oral malodor reduction.** | | | |
| **Composition** | **Baseline VSCin ppb*** | **1 Hour Post Film Application VSCin ppb** | **% Reduction of Malodor** |
| A | 390 | 290 | 28.0 |
| B | 520 | 490 | 7.0 |

| | | | |
|---|---|---|---|
| *ppb = parts per billion | | | |

## Claims

1. An orally consumable film composition for delivering breath freshening agents to the oral cavity which rapidly dissolves or disintegrates when applied in the oral cavity, the film composition being comprised of a homogeneous mixture of a protease enzyme, the enzyme being dispersed in a hydrophobic diluent or dispersant, and a water soluble or dispersible film forming polymer, wherein the enzyme is present in the film composition at a concentration of 0,1 to 5% by weight.

2. The film composition of claim 1 wherein the polymer is an hydroxyalkyl cellulose.

3. The film composition of claim 2 wherein the hydroxyakylcellulose is hydroxymethylpropyl cellulose.

4. The film composition of claim 1 wherein the water dispersible polymer is present at a concentration of 10 to 60% by weight.

5. The film composition of claim 1 wherein the protease enzyme is papain,

6. A use of an orally consumable film composition which rapidly dissolves or disintegrates in the oral cavity, the composition being comprised of a homogeneous mixture of a water soluble or dispersible polymer and a protease enzyme, the enzyme being dispersed in a hydrophobic diluent or dispersant, wherein the enzyme is present in the film composition at a concentration of 0.1 to 5% by weight for the manufacturing of a medicament for applying the film composition to the tongue of the user.

7. The use of claim 6 wherein the polymer is an hydroxyalkyl cellulose.

8. The use of claim 7 wherein the hydroxyalkylcellulose is hydroxymethylpropylcellulose.

9. The use of claim 6 wherein the water dispersible polymer is present at a concentration of 10 to 60% by weight.

10. The use of claim 6 wherein the protease enzyme is papain.

## Patentansprüche

1. Oral einnehmbare Filmzusammensetzung zur Verabreichung von Atemfrischemitteln in die Mundhöhle, die sich bei Verabreichung in der Mundhöhle rasch auflöst oder zerfällt, wobei die Folienzusammensetzung aus einer homogenen Mischung eines Proteaseenzyms, das in einem hydrophoben Verdünnungsmittel oder Dispergiermittel dispergiert ist, und eines wasserlöslichen oder dispergierbaren, filmbildenden Polymers besteht, wobei das Enzym in der Zusammensetzung in einer Konzentration von 0,1 bis 5 Gew.% vorhanden ist.

2. Filmzusammensetzung nach Anspruch 1, wobei das Polymer eine Hydroxyalkylcellulose ist.

3. Filmzusammensetzung nach Anspruch 2, wobei die Hydroxyalkylcellulose Hydroxymethylpropylcellulose ist.

4. Filmzusammensetzung nach Anspruch 1, in der das in Wasser dispergierbare Polymer in einer Konzentration von 10 bis 60 Gew.% vorhanden ist.

5. Filmzusammensetzung nach Anspruch 1, wobei das Proteaseenzym Papain ist.

6. Verwendung einer oral einnehmbaren Filmzusammensetzung, die sich in der Mundhöhle rasch auflöst oder zerfällt, wobei die Folienzusammensetzung aus einer homogenen Mischung eines wasserlöslichen oder dispergierbaren, filmbildenden Polymers und eines Proteaseenzyms besteht, das in einem hydrophoben Verdünnungsmittel oder Dispergiermittel dispergiert ist, wobei das Enzym in der Zusammensetzung in einer Konzentration von 0,1 bis 5 Gew.% vorhanden ist, zur Herstellung eines Medikaments zur Verabreichung der Filmzusammensetzung auf die Zunge des Nutzers.

7. Verwendung nach Anspruch 6, wobei das Polymer eine Hydroxyalkylcellulose ist.

8. Verwendung nach Anspruch wobei die Hydroxyalkylcellulose Hydroxymethylpropylcellulose ist.

9. Verwendung nach Anspruch 6, wobei das in Wasser dispergierbare Polymer in einer Konzentration von 10 bis 60 Gew.% vorhanden ist.

10. Verwendung nach Anspruch 6, wobei das Proteaseenzym Papain ist.

## Revendications

1. Composition de film consommable par voie orale pour délivrer des agents rafraîchisseurs d'haleine dans la cavité orale, qui se dissout ou se désintègre rapidement lorsqu'il est appliqué dans la cavité orale, la composition de film étant constituée d'un mélange homogène d'une enzyme protéase, l'enzyme étant dispersée dans un diluant ou dispersant hydrophobe, et d'un polymère filmogène soluble ou dispersible dans l'eau, l'enzyme étant présente dans la composition de film à une concentration de 0,1 à 5 % en poids.

2. Composition de film de la revendication 1, dans laquelle le polymère est une hydroxyalkylcellulose.

3. Composition de film de la revendication 2, dans laquelle l'hydroxyalkylcellulose est l'hydroxyméthylpropylcellulose.

4. Composition de film de la revendication 1, dans laquelle le polymère dispersible dans l'eau est présent à une concentration de 10 à 60 % en poids.

5. Composition de film de la revendication 1, dans laquelle l'enzyme protéase est la papaïne.

6. Utilisation d'un film consommable par voie orale qui se dissout ou se désintègre rapidement dans la cavité orale, la composition étant constituée d'un mélange homogène d'un polymère soluble ou dispersible dans l'eau et d'une enzyme protéase, l'enzyme étant dispersée dans un diluant ou dispersant hydrophobe, l'enzyme étant présente dans la composition de film à une concentration de 0,1 à 5 % en poids pour la fabrication d'un médicament, pour appliquer la composition de film sur la langue de l'utilisateur.

7. Utilisation de la revendication 6, dans lequel le polymère est une hydroxyalkylcellulose.

8. Utilisation de la revendication 7, dans lequel l'hydroxyalkylcellulose est l'hydroxyméthylpropylcellulose.

9. Utilisation de la revendication 6, dans lequel le polymère dispersible dans l'eau est présent à une concentration de 10 à 60 % en poids.

10. Utilisation de la revendication 6, dans lequel l'enzyme protéase est la papaïne.
